# EUROPEAN PATENT SPECIFICATION

(11) **EP 2 758 524 B1**
(45) Date of publication and mention of the grant of the patent: **26.12.2018**
(21) Application number: 12766635.2
(22) Date of filing: 24.09.2012
(51) Int. Cl.: C12N 5/077, C12N 5/10, C12N 15/867, C07K 14/15, C07K 14/005, C07K 14/025

(54) **FIBROBLASTS CELLULAR MODEL FOR ASSESSING EFFICACY OF CANCER TREATMENTS BY SHH/PTCH PATHWAY ANTAGONISTS**
ZELLULÄRES FIBROBLASTENMODELL ZUR BEURTEILUNG DER WIRKSAMKEIT VON KREBSBEHANDLUNGEN DURCH ANTAGONISTEN DES SHH/PTCH-SIGNALWEGES
MODÈLE CELLULAIRE DE FIBROBLASTES POUR L'ESTIMATION DE L'EFFICACITÉ DE TRAITEMENTS ANTICANCÉREUX PAR DES ANTAGONISTES DE LA VOIE SHH/PTCH

(30) Priority: 23.09.2011 US 201161538586 P
(43) Date of publication of application: 30.07.2014
(73) Proprietor: Galderma Research & Development, 06410 Biot (FR); CNRS, 75794 Paris Cedex 16 (FR)
(72) Inventor: BURTY, Elodie, 69210 Sourcieux-les-Mines (FR); MAGNALDO, Thierry, 06100 Nice (FR); GACHE, Yannick, 06300 Nice (FR)
(74) Representative: Starck-Loudes, Anne-Caroline
(86) International application number: PCT/EP2012/068777
(87) International publication number: WO 2013/041725

(56) References cited:
- WO-A1-2010/015618
- US-B1- 6 458 593
- SHIMADA T ET AL: "LIFESPAN EXTENSION OF BASAL CELL NEVUS SYNDROME FIBROBLASTS BY TRANSFECTION WITH MOUSE PRO OR V-MYC GENES", INTERNATIONAL JOURNAL OF CANCER, vol. 39, no. 5, 1987, pages 649-655, XP009165317, ISSN: 0020-7136
- MAJMUDAR G ET AL: "Increased expression of matrix metalloproteinase-3 (stromelysin-1) in cultured fibroblasts and basal cell carcinomas of nevoid basal cell carcinoma syndrome", MOLECULAR CARCINOGENESIS, ALAN LISS, NEW YORK, NY, US, vol. 11, no. 1, 1 September 1994 (1994-09-01), pages 29-33, XP002506350, ISSN: 0899-1987, DOI: 10.1002/MC.2940110106
- VALIN ALEXANDRE ET AL: "PTCH1(+/-) Dermal Fibroblasts Isolated from Healthy Skin of Gorlin Syndrome Patients Exhibit Features of Carcinoma Associated Fibroblasts", PLOS ONE, vol. 4, no. 3, March 2009 (2009-03), XP009165727, ISSN: 1932-6203
- F. BRELLIER ET AL: "Ultraviolet responses of Gorlin syndrome primary skin cells", BRITISH JOURNAL OF DERMATOLOGY, vol. 159, no. 2, 1 August 2008 (2008-08-01), pages 445-452, XP055044573, ISSN: 0007-0963, DOI: 10.1111/j.1365-2133.2008.08650.x
- VALIN ALEXANDRE ET AL: "[Dermal fibroblasts exert a key influence in the development of basal-cell skin cancers: the model of Gorlin syndrome].", MÉDECINE SCIENCES : M/S JAN 2010, vol. 26, no. 1, January 2010 (2010-01), pages 22-25, XP009165729, ISSN: 0767-0974

## Description

The present invention is in the domain of pharmacy and more specifically in skin cancer area and particular for Basal Cell Carcinoma (BCC). The present invention provides a cellular model targeting the Sonic Hedgehog/Patched (SHH/PTCH) pathway dysregulation or inappropriately activated as well as screening method using this cellular model to screen pharmacological compounds able to treat or prevent BCC lesions.

The Hedgehog pathway is normally active during embryonic development and plays a central role in cell differentiation and proliferation.

Inappropriate activation or dysregulation of the Hedgehog pathway is believed to play a critical role in the proliferation and survival of certain cancer cells, including in basal cell carcinoma and medulloblastoma.

Known pathway-activating mutations include those that impair the ability of PTCH, a transporter-like Hh receptor, to restrain Smoothened (SMO) activation of transcriptional targets via the GLI family of latent transcription factors.

Binding of Hh ligand to PTCH is functionally equivalent to genetic loss of PTCH, in that pathway activation by either requires activity of SMO, a seven transmembrane protein that binds to and is inactivated by the pathway antagonist, cyclopamine.

The implication of PATCHED pathway activation in several cancer conditions, most notably in BCCs, has motivated much effort to set up experimental systems to assess the inhibitory activity of small molecules.

The existing systems to measure activation or inhibition of the activated SHH/PTCH pathway, are based on cell lines from human or mouse origin. These cells can schematically be classified in two categories destined to measure i-) endogenous cellular events after treatment; these events include triggering of a differentiation process and modulation of gene expression, notably of those genes known as transcriptional targets of pathway activation; ii-) cell lines engineered to report pathway activation / inhibition after transient or permanent introduction of reporter constructs made of responsive DNA driving a reporter gene. Cell lines developed so far are:
- Human normal primary keratinocytes and fibroblasts in reconstructed skin where expression of GLI 1 and GLI2 mRNA have been measured to demonstrate inhibition by the small Robotnikinin molecule of SHH/PTCH pathway activation by SHH (Stanton et al, 2009)
- Healthy human primary keratinocytes from patients with nevoid bas3al cell carcinoma or Gorlin syndrome have been isolated to mimic the somatic loss of one PATCHED allele in sporadic BCC epidermal cells (Brellier et al, 2008a).

However, the above described cell lines have some disadvantages. Most of cell lines are not stable in the sense that after several passages the expression of inserted genes decreases strongly or is shut down. Either those cell lines are not sufficiently robust to be efficient and sensitive to be used in a drug screening as a model. Shimada T et al. (Lifespan extension of basal cell nevus syndrome fibroblasts by transfection with mouse pro or v-myc genes, International journal of cancer, vol. 39, no.5, 1987, pages 649-655) describe partially immortalized and anchorage-dependent transfected BCNS fibroblasts. Majmudar G et al. [Increased expression of matrix metalloproteinase-3 (stromelysin-1) in cultured fibroblasts and basal cell carcinomas of nevoid basal cell carcinoma syndrome, Molecular carcinogenesis, Alan Liss, New York, NY, US, Vol. 11, no. 1, 1 September 1994 (1994-09-01), pages 29-33] examine the expression of matrix metalloproteinase-3 in BCC tumor specimen, adjacent normal skin, and fibroblast isolated from the normal-appearing skin of NBCCS patients. None of the cited prior art provides a system to allow a simple detection of activation in human skin cells, including keratinocytes and fibroblasts.

Thus, there is a need for developing a human cell line easy to produce and to use, efficient, being a relevant model and sensitive for the screening or assessment of molecules libraries.

The inventors have developed a new cell line providing strong advantages. Indeed, the present description provides an immortalized cell line of human skin fibroblasts which responds to pathway stimulation without the need to introduce an exogenous reporter cassette using genetic engineering, thus that cell line is characterized in that reporter gene is endogenous with its own elements of response to pathway activation. The reporter capacity of this human cell line is stable overtime, particularly even after a high rate from passages in tissue culture.

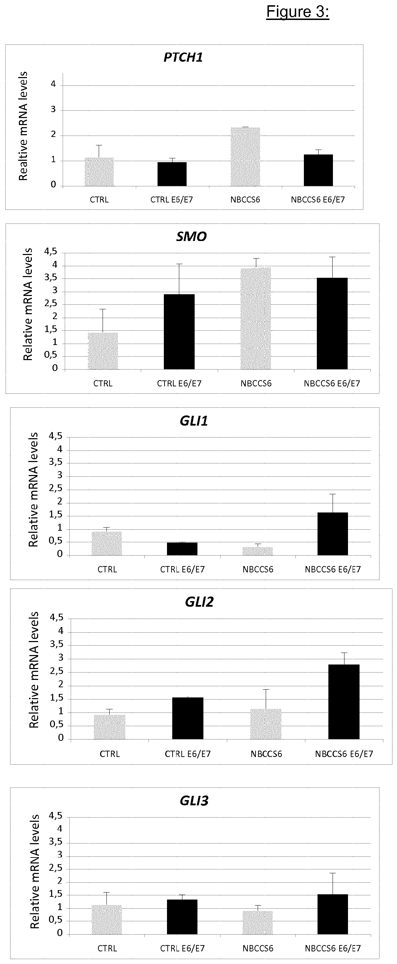

Herein described is an immortalized reporter fibroblast cell line which expresses the PTCH protein and/or other members of the pathway that are necessary to convey responses to agonists and antagonists of the said pathway as shown in figure 3. Said cell line is immortalized by retroviral transduction of pLE6/E7SN. In addition, this cell line is produced in standard cell culture medium without addition of sophisticated goodies but only ready to use elements.

The description provides also a process for obtaining immortalized human fibroblasts as described above, comprising the following steps:
- isolate human primary Fibroblasts from healthy skin from individual having NBCCS or Gorlin syndrome
- immortalize the cell line by retroviral transduction with pLE6/E7SN
- select immortalized cell line with a medium of selection
- check for attenuation of P53 expression
- check for growth properties,

Also herein described is a drug screening method, wherein said immortalized fibroblast cell line as described above is used to screen. In a preferred aspect herein described, the drug screening method comprises the following steps:
a). bringing one sample of immortalized fibroblasts cell line as described above into contact with one or more of the test compounds;
b). measuring the expression of endogenous reporter gene expression, namely by quantitative measurement of accumulation of the GLI 1 mRNA , preferentially by Q- RT-PCR;
c). selecting the compounds for which a modulation of the expression of the activity of endogenous reporter gene is measured in b) and compared with no drug mixture.

In a preferred embodiment, the drug identified and/or selected according to the drug screening method as described above is an anti-tumor drug.

Further herein described is an in vitro method for the screening of candidate compounds for the preventive and/or curative treatment of cutaneous cancer and preferentially basal cell carcinoma.

The description regards also to a drug obtainable with the drug screening method as described above.

### Detailed description

Basal Cell Carcinomas (BCC) of the skin is the commonest human cancer. BCCs derive from epidermal keratinocytes. The great majority of BCCs occurs on photo-exposed skin due to ultraviolet induced mutagenesis. The steadily rising incidence of BCCs in the last decades is attributed to increasing enthusiasm for recreational sun exposure. Although BCC rarely metastasize, they can result in local destruction and invasion of underlying tissues and consequently, life threatening complications. BCC are usually treated by local surgical excision, topical chemotherapy, photodynamic therapy, but, according to tumor size, location and frequency, there may be considerable aesthetic sequelae. Thus, drawbacks of current BCC treatments strongly support the need for pharmacological innovations that should specifically target the SONIC in so far as inappropriate and constitutive activation of this pathway is associated with the vast majority of BCC (see below). In several cancers including BCCs, It has been suggested that accumulation of mRNA corresponding to target genes of the pathway depends on interactions of tumor cells with fibroblasts of the microenvironment. Furthermore, molecules that target the SHH pathway could also be of interest in the treatment of other / non-BCC cancer conditions where the influence of stromal microenvironment (e. g. melanoma, pancreas, esophagus, liver, prostate, lung, muscle, colon) is thought to play a role in inappropriate activation of the SHH/PTCH pathway (for revue see, (Scales and de Sauvage, 2009)).

### The SHH/ PATCHED pathway

The SHH/PTCH signaling pathway is essential during embryogenesis and development where it controls cell fate by modulating proliferation and differentiation. Animal models, notably the fruit fly drosophila melanogaster, have shown that at specific stages of development, some cells produce and emit a signal, the Hedgehog molecule (HH), which, in turn, is received by target cells. In vertebrates, the family of Hedgehog molecules is composed of Sonic Hedgehog, SHH, Desert Hedgehog, DHH, and Indian Hedgehog, IHH.

Target cells (of these ligands) express PATCHED (PTCH), a putative twelve pass transmembrane protein acting as the receptor of HH molecules. When HH molecules are not expressed and/or not secreted at the vicinity of target cells, PTCH acts as a repressor of the pathway by inhibiting another transmembrane protein called SMOTHENED (SMO). SMO is a putative seven pass transmembrane protein apparented to G-protein coupled receptors. The inhibition of SMO by PTCH is relieved in the presence of HH molecules bound to PTCH. Derepression of SMO leads to activation of transcription factors of the GLI family (named GLI1, 2 and 3) that activate (GLI1 and 2) or repress (GLI3), the transcription of their target genes. Interestingly, PTCH1 is a transcriptional target of GLI1 and GLI2 factors (Scales and de Sauvage, 2009).

The importance of the SHH / PTCH pathway is illustrated by severe diseases due to mutations affecting its integrity at different levels. Notably, in the human, heterozygous mutations in the PTCH1 gene are responsible for the dominantly inherited genetic syndrome called nevoid basal cell carcinoma syndrome (NBCCS or Gorlin syndrome). NBCCS patients are highly prone to BCCs that generally (about 50 % cases) present with a loss of heterozygosity in the PTCH1 locus. In Gorlin patients, more than 50% BCCs also bear mutation in the tumor suppressor gene TP53, suggesting some cooperation of the P53 and the SHH/PTCH pathways toward development of BCCs. Very interestingly, the two *PTCH1* alleles are also lost in most sporadic (general population) BCCs; in the latter case, again, the two TP53 alleles are found mutated in 10 to 50% sporadic BCCs. 20-30 % sporadic BCCs are mutated in both TP53 and PTCH1. In both NBCSS and sporadic BCCs, inactivation of PTCH results in constitutive activation of the pathway with accumulation GLI1 and GLI2 mRNAs (Dahmane *et al,* 1997; Unden *et al,* 1996). In contrast to sporadic BCCs that almost exclusively develop in photo exposed skin area, about 40% NBCCS BCCs develop in non-exposed skin. In addition, our previous observations have indicated that primary NBCCS primary fibroblasts isolated from healthy skin expressed a transcriptome resembling that characterized in fibroblasts associated to sporadic carcinomas (CAF) (Valin et al, 2009 ; Valin and Magnaldo, 2008). Together, these observations strongly support the idea of a strong contribution of dermal fibroblasts in carcinoma development in NBCCS patients.

The implication of the SHH/PTCH pathway activation in several cancer conditions, most notably in BCCs, has motivated much effort to set up experimental systems to assess the inhibitory activity of small molecules.

The existing systems of activity measure are based on cell lines from human or mouse origin. These cells can schematically be classified in two categories destined to measure i-) endogenous cellular events after treatment; these events include triggering of a differentiation process and modulation of gene expression, notably of those genes known as transcriptional targets of pathway activation; ii-) cell lines engineered to report pathway activation / inhibition after transient or permanent introduction of reporter constructs made of responsive DNA driving a reporter gene. Cell lines developed so far in the prior art reveals that none of those system allows simple detection of activation in human epidermal keratinocytes.

To provide a simple detection system, the inventors have worked to develop a human cell line in respect of the following specifications (i.e. what we need for easy, efficient, relevant, sensitive assessment of molecules libraries):
- human cells,
- skin cells,
- growth in standard medium,
- needing no feeders,
- genetic stability and activity of the reporter gene a long period of time, including long term expression over cell generations;
- highly sensitive to activation; the cell line must report activation at doses closed to ligand (SHH) affinity, thus at the nM order.

To fulfil these specifications, the strategy was to use a human cell strain derived from healthy individuals having NBCCS or Gorlin syndrome. The choice of NBCCS cell was dictated by the fact that our previous investigations have suggested that NBCCS (heterozygotes for the PATCHED, suggesting the partial loss of the PTCH repressor activity) cells are particularly sensitive to pathway activation. Thus, we hypothesized that cells from those NBCCS patients could certainly constitute a valuable tool to measure activation and inhibition of the activation with a high sensitivity. As indicated in prior art, no such line is available, neither from academic nor from commercial sources. The rationale of using natural endogenous responses to SHH-like agonists stems from i-) the physiological relevance, i.e. good sensitivity of the cell line relying on natural elements of transcriptional control (control regions of SHH-regulated genes), ii-) avoiding the use of direct tandem repeats of GliBS (n=8) upstream the Firely luciferase gene as described (Sasaki et al, 1997). Direct tandem repeats of GliBS are known to be very unstable; as in many other laboratories, all attempts to construct a reporter cell line using these sequences have failed. Concerning the easiness of growth in standard non sophisticated culture media, we decided to abrogate or at least to attenuate, the expression of the tumor suppressor gene TP53.

In the specific case of NBCCS cells, other advantages of abrogating P53 stem from our and others reports indicating that: i-) P53 stabilization after a single UVB irradiation is higher and prolonged in NBCCS compared to control cells (Brellier et al, 2008b); ii-) there is a mutual inhibition of GN1 and P53 (Stecca and Ruiz i Altaba, 2009); iii-) 20-30 % BCCs bear mutations in both TP53 and PATCHED suggesting that attenuation of P53 could enhance sensitivity to PTCH pathway activators.

Thus, it is reasoned that abrogation or attenuation of the P53 pathway using E6-E7 oncogenic proteins of Human Papilloma Virus 16 (HPV16) would favor activation of the SHH/PTCH pathway in human skin cells, including fibroblasts. Preferably, it was decided to use human primary fibroblasts from healthy skin (non-photo exposed non tumoral) from individuals having NBCCS or Gorlin syndrome, after transformation with the E6-E7 oncogenic proteins (NBCCS 6 E6/E7 fibroblasts).

Herein described is thus an immortalized cell line of human fibroblasts from healthy individuals having NBCCS or Gorlin syndrome expressing an enhanced and stable response toward PTCH pathway activation overtime, particularly even after a high rate from passages in tissue culture.

It meant by stable expression of the endogenous reporter gene overtime that after high number of passages the level expression is the same as the initial level without recombination or loss of chromosomal material, as this can be observed in cell lines that express an exogenous reporter gene (i.e. Luciferase) under the control of GLI binding sequence in direct repeat (X8) tandem configuration (Sasaki et al, 1997).

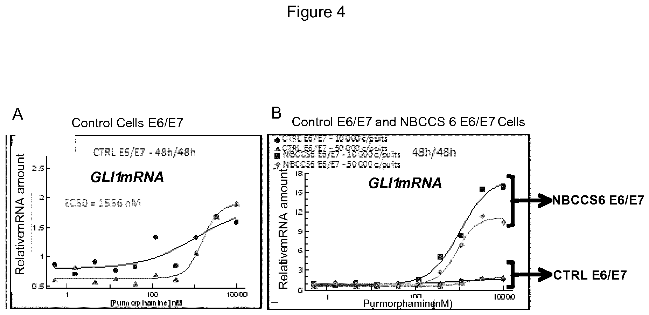

The inventors have shown that the immortalized fibroblasts from healthy individuals having NBCCS or Gorlin syndrome cell line as herein described express the PTCH protein and/or other members of the pathway that are necessary to convey responses to agonists and antagonists of the said pathway as shown in figure 4. Said cell line is immortalized by retroviral transduction. The skilled in the art is familiar with retroviral transduction techniques and all of them are applicable to the present invention. Any kind of retrovirus can be used such as Moloney murine leukemia virus (MoMLV), lentivirus, Eptein- Barr virus (EBV).... MoMLV is preferred for high performance of infection in human primary fibroblasts (Quilliet et al, 1996). The retroviral transduction of pLE6/E7SN is preferred in this context.

In addition, this cell line is produced in standard culture medium (DMEM based containing non sophisticated, ready to use goodies). In addition the cell line stands temporary serum starvation (0.5 % serum) which allows avoiding interference with the activity response. Thus, response of cells to pathway activation in a quasi-defined medium providing the advantage of growing cells in medium which does not interfere with activity response. The invention provides thus a robust model with expected or calibrated response which avoids any interfering factors.

Also herein described is a drug screening method, wherein said immortalized fibroblast cell line as described above is used to screen. The description relates to an in vitro screening method of the PTCH pathway inhibitors for treating skin cancer and preferably BCC, comprising determining the capacity of said drug to inhibit or down regulate expression or biological activity of the PTCH pathway.

In a preferred aspect herein described, the drug screening method comprises the following steps:
a). bringing one sample of immortalized cell line as described above into contact with one or more of the test compounds;
b). measuring the expression or the activity of the reporter gene mRNA, preferably GLI1
c). selecting the compounds for which a modulation of the expression of the reporter gene, preferably GLI1 thereof, is measured in b) and compared with no drug mixture.

In the context of the invention, any other gene responsive to the pathway activation (PATCHED1, CCD1, BCL2, SNAIL etc.) known by the skilled artisan is applicable. In a preferred embodiment, the reporter gene is GLI1.

The present description provides tools for selecting SHH/PTCH pathway modulators. Those modulators are activators or inhibitors.

In a preferred embodiment, the drug identified and/or selected according to the drug screening method as described above is an anti-tumor drug. The reporter gene, preferably GLI1, is first activated and inhibition efficacy of one or several drug candidates (isolated or in a mixture) is assessed, preferably with increasing concentration. The examples provide an illustration with a particular embodiment in GLI 1 mRNA accumulation as reporter model.

### FIGURES

The following figures illustrate the invention:
**Figure 1****:** Schematic map of the LE6E7 SN proviral construct. LTR5', long terminal repeat 5'. E6E7, sequence of the human papilloma virus 16 encoding the E6 and E7 transforming proteins. Neo, neomycin phosphotransferase gene confering resistance to G418 antibiotic. LTR 3', long terminal repeat 3'.
**Figure 2****:** Transformation of the control or NBCCS6 fibroblasts cell line by the E6/E7 oncoproteins
**Figure 3****:** RT-Q-PCR analysis of expression of mRNAs encoding actor proteins of the SHH/PTCH_pathway_shows that NBCCS 6 E6/E7 fibroblasts express essential actors of the PTCH/SHH pathway
**Figure 4****:** Comparative Q-PCR analyses of GLI1 mRNA accumulation after treatment of fibroblats cell lines with purmorphamin. A, CTRL E6/E7 cells, B, representation of the response of CTRL cell (shown in A) to the agonist in comparision to the response of NBCS E6/E7 cells. Note that NBCCS 6 E6/E7 exhibit a 7 x times higher response than CTRL cells in the same experimental conditions.

### EXAMPLES

The examples which follow illustrate the invention without limiting the scope thereof.

### Example 1 : Materials and Methods

### Cell Culture

Human primary fibroblasts (named CTRL or NBCCS) were isolated from a healthy non photo-exposed skin biopsy of either a control patient or a patient with caracteristic NBCCS after informed consent (Otto et al, 1999; Valin et al, 2009)

### Cell transformation and selection

E6/E7 cells. Note that NBCCS 6 E6/E7 exhibit a 7 x times higher response than CTRL cells in the same experimental conditions.

### EXAMPLES

The examples which follow illustrate the invention without limiting the scope thereof.

### Example 1: Materials and Methods

### Cell Culture

Human primary fibroblasts (named CTRL or NBCCS) were isolated from a healthy non photo-exposed skin biopsy of either a control patient or a patient with caracteristic NBCCS after informed consent (Otto et al., 1999; Valin et al., 2009)

### Cell transformation and selection

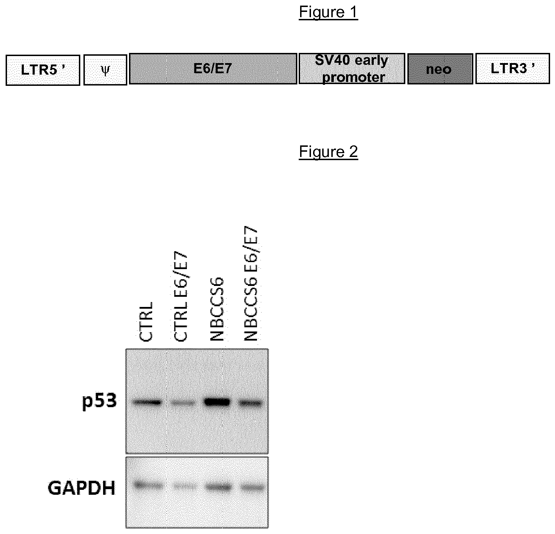

The CTRL and NBCCS6 cell lines were then immortalized by retroviral transduction with pLE6/E7SN resulting in CTRL and NBCCS-E6-E7 (figure 1) (Halbert et al., 1991, 1992). Cells were grown at 37 °C in a humified atmosphere containing 5 % CO2, DMEM medium, 50 U/ml penicillin, 50 µg/ml streptomycin, 0.125 µg/ml amphotericin B, 2mM L-Glutamine, 1 mM Sodium pyruvate, 1x non essential amino acids.

G418 is an aminoglycoside antibiotic similar in structure to gentamicin B1, produced by *Micromonospora rhodorangea.* G418 blocks polypeptide synthesis by inhibiting the elongation step in both prokaryotic and eukaryotic cells. Resistance to G418 is conferred by the **Neomycin resistance gene (*neo*)** from Tn5 encoding an aminoglycoside 3'-phosphotransferase, APH 3' II.

Selection in mammalian cells is usually achieved in three to seven days with concentrations ranging from 200 to 1000 µg/ml (Arnaudeau-Bégard et al., 2003).

### Real time quantitative PCR

Total RNA was extracted with RNeasy® Mini kit according to manufacturer's instructions (Qiagen, Hilden, Germany). Reverse transcription was performed on 1 µg total RNA with Superscript II Reverse Transcriptase (Roche Applied Science, Basel, Switzerland) by using random primers. Q-PCR was carried out using the 7900 HT Fast Real-Time PCR System (Applied Biosystems, Foster City, CA, USA).

### Example 2: Transformation of the control or NBCCS6 fibroblasts cell line by the E6/E7 oncoproteins

Effective transformation of the CTRL and NBCCS6 cell line was assessed by analysing their growth properties and attenuation of expression of the P53 tumor suppressor protein. Figure 2 shows a western blot analysis of P53 in cell extracts prepared from preconconfluent (about 80 %) primary fibroblasts (CTRL, or NBCCS6), before or after transformation using the E6E7 encoding retroviral vector (CTRL E6/E7, NBCCS6 E6/E7), The western blot (figure 2) shows the drastic decrease in the amount of the P53 protein. GAPDH is a control of loading.

### Example 3:RT-Q-PCR analysis of expression of mRNAs encoding actor proteins of the SHH/PTCH_pathway shows that NBCCS 6 E6/E7 fibroblasts express essential actors of the PTCH/SHH pathway

As shown in figure 3, NBCCS 6 E6/E7 fibroblasts express essential actors of the PTCH/SHH pathway.
RT-Q-PCR was performed from total cDNAs prepared from the indicated cells. CTRL , human primary dermal fibroblasts before or after CTRL E6/E7 immortalization by pLE6E7 retroviral transduction. NBCC6, primary fibroblast from Nevoid Basal Cell Carcinoma Syndrome patient # 6 before or after (NBCCS6 E6/7) immortalization by pLE6E7 retroviral transduction.

### Example 4: Comparative Q-PCR analyses of GLI1 mRNA accumulation after treatment of fibroblats cell lines with purmorphamin.,

To evaluate small Molecules Modulators of the SHH/PTCH pathway, the Purmorphamine (SMO agonist) was used. Purmorphamine (SMO agonist) was diluted in DMSO at stock concentrations of 50 mM and 10 mM, respectively. To avoid side effects and toxicity, the final concentration of DMSO was fixed to 0.1 % DMSO.

As shown in figure 4, the comparative Q-PCR analyses of GLI1 mRNA accumulation after treatment of fibroblats cell lines with purmorphamin are represented in figure 4A, CTRL E6/E7 cells,
Figure 4B, is the representation of the response of CTRL cell (shown in A) to the agonist in comparison to the response of NBCS_E6/E7 cells.

It should be noted that NBCCS 6 E6/E7 exhibit a 7 x times higher response than CTRL cells in the same experimental conditions.

### References

Arnaudeau-Bégard, C., Brellier, F., Chevallier-Lagente, O., Hoeijmakers, J.H., Bernerd, F., Sarasin, A., and Magnaldo, T. (2003). Genetic correction of DNA repair deficient/cancer prone xeroderma pigmentosum group C keratinocytes. Hum Gene Ther 14, 983-996.
Brellier, F., Bergoglio, V., Valin, A., Barnay, S., Chevallier-Lagente, O., Vielh, P., Spatz, A., Gorry, P., Avril, M.F., and Magnaldo, T. (2008a). Heterozygous mutations in the tumor suppressor gene PATCHED provoke basal cell carcinoma-like features in human organotypic skin cultures. Oncogene 27, 6601-6606.
Brellier, F., Valin, A., Chevallier-Lagente, O., Gorry, P., Avril, M.F., and Magnaldo, T. (2008b). Ultraviolet responses of Gorlin syndrome primary skin cells. Br J Dermatol 159, 445-452.
Dahmane, N., Lee, J., Robins, P., Heller, P., and Ruiz i Altaba, A. (1997). Activation of the transcription factor Gli1 and the Sonic hedgehog signalling pathway in skin tumours [published erratum appears in Nature 1997 Dec 4;390(6659):536]. Nature 389, 876-881.
Halbert, C.L., Demers, G.W., and Galloway, D.A. (1991). The E7 gene of human papillomavirus type 16 is sufficient for immortalization of human epithelial cells. J Virol 65, 473-478.
Halbert, C.L., Demers, G.W., and Galloway, D.A. (1992). The E6 and E7 genes of human papillomavirus type 6 have weak immortalizing activity in human epithelial cells. J Virol 66, 2125-2134.
Otto, A.I., Riou, L., Marionnet, C., Mori, T., Sarasin, A., and Magnaldo, T. (1999). Differential behaviors toward ultraviolet A and B radiation of fibroblasts and keratinocytes from normal and DNA-repair-deficient patients. Cancer Res 59, 1212-1218.
Quilliet, X., Chevallier-Lagente, O., Eveno, E., Stojkovic, T., Destee, A., Sarasin, A., and Mezzina, M. (1996). Long-term complementation of DNA repair deficient human primary fibroblasts by retroviral transduction of the XPD gene. Mutat Res 364, 161-169.
Sasaki, H., Hui, C., Nakafuku, M., and Kondoh, H. (1997). A binding site for Gli proteins is essential for HNF-3beta floor plate enhancer activity in transgenics and can respond to Shh in vitro. Development 124, 1313-1322.
Scales, S.J., and de Sauvage, F.J. (2009). Mechanisms of Hedgehog pathway activation in cancer and implications for therapy. Trends Pharmacol Sci 30, 303-312.
Stanton, B.Z., Peng, L.F., Maloof, N., Nakai, K., Wang, X., Duffner, J.L., Taveras, K.M., Hyman, J.M., Lee, S.W., Koehler, A.N., et al. (2009). A small molecule that binds Hedgehog and blocks its signaling in human cells. Nat Chem Biol 5, 154-156.
Stecca, B., and Ruiz i Altaba, A. (2009). A GLI1-p53 inhibitory loop controls neural stem cell and tumour cell numbers. EMBO J 28, 663-676.
Unden, A.B., Holmberg, E., Lundh-Rozell, B., Stahle-Backdahl, M., Zaphiropoulos, P.G., Toftgard, R., and Vorechovsky, I. (1996). Mutations in the human homologue of Drosophila patched (PTCH) in basal cell carcinomas and the Gorlin syndrome: different in vivo mechanisms of PTCH inactivation. Cancer Res 56, 4562-4565.
Valin, A., Barnay-Verdier, S., Robert, T., Ripoche, H., Brellier, F., Chevallier-Lagente, O., Avril, M.F., and Magnaldo, T. (2009). PTCH1 +/- dermal fibroblasts isolated from healthy skin of Gorlin syndrome patients exhibit features of carcinoma associated fibroblasts. PLoS One 4, e4818.
Valin, A., and Magnaldo, T. (2008). Method for determining a predisposition to basal cell carcinoma and for screening treatments therof. Brevet européen, depose le 05 Aout 2008, EP 08 3054486.

## Claims

1. An immortalized cell line of human fibroblasts derived from the healthy skin of an individual having nevoid basal cell carcinoma syndrome (NBCCS or Gorlin syndrome), said cell line having been immortalized by retroviral transduction with a retrovirus expressing the E6 and E7 proteins of human papilloma virus 16 (HPV16), and said immortalized cell line stably expressing GLI1 as an inducible endogenous reporter gene of the SHH/PTCH-pathway activation over cell generations.

2. The immortalized cell line of human fibroblasts according to claim 1, **characterized in that** it expresses PTCH.

3. The immortalized cell line of human fibroblasts according to claim 1, **characterized in that** it expresses PTCH1, SMO, GLI1, GLI2 and GLI3.

4. The immortalized cell line of human fibroblasts according to claim 1, **characterized in that** it is produced without using feeder cells.

5. A process for obtaining immortalized human fibroblasts as described in anyone of claims 1 to 4, comprising the following steps:
- using human primary fibroblasts isolated from the healthy skin of an individual having NBCCS or Gorlin syndrome,
- immortalizing cell line by retroviral transduction with a retrovirus expressing the E6 and E7 proteins of HPV16,
- selecting cell line expression with a medium of selection,
- checking for attenuation of p53 expression, and
- checking for growth properties.

6. A drug screening method, wherein a immortalized cell line of human fibroblasts according to anyone of claims 1 to 5 is used.

7. The drug screening method according to claim 6, comprising the following steps of:
a. contacting one sample of immortalized cell line of human fibroblasts according to anyone of claims 1 to 4 with one or more test compounds or with a mixture of compounds,
b. measuring the expression or the activity of the GLI1 reporter gene, and
c. selecting the compounds for which a modulation of the expression of the GLI1 reporter gene is measured in b) when compared to the expression in the absence of any test compounds or mixture of compounds.

8. The drug screening method according to claim 7, wherein the identified drug is an antitumor drug.

9. An *in vitro* method for screening candidate compounds for use for the preventive and/or curative treatment of cutaneous cancer, comprising the following steps of:
a. contacting a immortalized cell line of human fibroblasts according to anyone of claims 1 to 4 with one or more test compounds or with a mixture of compounds,
b. measuring the expression or the activity of the GLI1 reporter gene, and
c. selecting the compounds for which a modulation of the expression or of the activity of the GLI1 reporter gene is measured in b) when compared to the expression or activity in the absence of any test compounds or mixture of compounds.

## Patentansprüche

1. Eine immortalisierte Zelllinie von humanen Fibroblasten, die von der gesunden Haut eines Individuums mit nävalem Basalzellkarzinom-Syndrom (NBCCS oder Gorlin-Syndrom) abgeleitet ist, wobei die Zelllinie durch retrovirale Transduktion mit einem Retrovirus, der die E6- und E7-Proteine des humanen Papillomavirus 16 (HPV16) exprimiert, und wobei die immortalisierte Zelllinie GLI1 als induzierbares endogenes Reportergen der Aktivierung des SHH/PTCH-Wegs über Zellgenerationen stabil exprimiert.

2. Immortalisierte Zelllinie von humanen Fibroblasten nach Anspruch 1, **dadurch gekennzeichnet, dass** sie PTCH exprimiert.

3. Immortalisierte Zelllinie von humanen Fibroblasten nach Anspruch 1, **dadurch gekennzeichnet, dass** sie PTCH1, SMO, GLI1, GLI2 und GLI3 exprimiert.

4. Immortalisierte Zelllinie von humanen Fibroblasten nach Anspruch 1, **dadurch gekennzeichnet, dass** sie ohne Verwendung von Feeder-Zellen hergestellt wird.

5. Ein Verfahren zum Erhalten immortalisierter humaner Fibroblasten nach einem der Ansprüche 1 bis 4, umfassend die folgenden Schritte:
- Verwenden von humanen primären Fibroblasten, die aus der gesunden Haut eines Individuums mit NBCCS- oder Gorlin-Syndrom isoliert wurden,
- Immortalisieren der Zelllinie durch retrovirale Transduktion mit einem Retrovirus, der die Proteine E6 und E7 von HPV16 exprimiert,
- Selektionieren des Zelllinienexpression mit einem Selektionsmedium,
- Überprüfen der Abschwächung der p53 Expression und
- Überprüfen der Wachstumseigenschaften.

6. Arzneimittelscreening-Verfahren, wobei eine immortalisierte Zelllinie von humanen Fibroblasten nach einem der Ansprüche 1 bis 5 verwendet wird.

7. Arzneimittelscreening-Verfahren nach Anspruch 6, umfassend die folgenden Schritte:
a. In Kontakt Bringen einer Probe der immortalisierten Zelllinie von humanen Fibroblasten nach einem der Ansprüche 1 bis 4 mit einer oder mehreren Testverbindungen oder mit einem Gemisch von Verbindungen,
b. Messen der Expression oder Aktivität des GLI1-Reportergens und
c. Auswählen der Verbindungen, für die eine Modulation der Expression des GLI1-Reportergens in b) gemessen wird, verglichen mit der Expression in Abwesenheit von irgendwelchen Testverbindungen oder einem Gemisch von Verbindungen.

8. Arzneimittelscreening-Verfahren nach Anspruch 7, wobei das identifizierte Arzneimittel ein Antitumorarzneimittel ist.

9. In-vitro-Verfahren zum Screenen von Kandidatenverbindungen zur Verwendung für die präventive und/oder kurative Behandlung von Hautkrebs, umfassend die folgenden Schritte:
a. In Kontakt Bringen einer immortalisierten Zelllinie von humanen Fibroblasten nach einem der Ansprüche 1 bis 4 mit einer oder mehreren Testverbindungen oder mit einem Gemisch von Verbindungen,
b. Messen der Expression oder Aktivität des GLI1-Reportergens und
c. Auswählen der Verbindungen, für die eine Modulation der Expression oder der Aktivität des GLI1 -Reportergens in b) gemessen wird, verglichen mit der Expression oder Aktivität in Abwesenheit von irgendwelchen Testverbindungen oder einem Gemisch von Verbindungen.

## Revendications

1. Lignée cellulaire immortalisée de fibroblastes humains dérivée de la peau saine d'un individu présentant un syndrome de carcinome baso-cellulaire nevoïde (NBCCS ou syndrome de Gorlin), ladite lignée cellulaire ayant été immortalisée par transduction rétro virale avec un rétrovirus exprimant les protéines E6 et E7 du virus du papillome humain 16 (HPV16), et ladite lignée cellulaire immortalisée exprimant de manière stable GLI1 en tant que gène rapporteur endogène inductible de l'activation de la voie SHH/PTCH à travers les générations cellulaires.

2. Lignée cellulaire immortalisée de fibroblastes humains selon la revendication 1, **caractérisée en ce qu'**elle exprime le PTCH.

3. Lignée cellulaire immortalisée de fibroblastes humains selon la revendication 1, **caractérisée en ce qu'**elle exprime PTCH1, SMO, GLI1, GLI2 et GLI3.

4. Lignée cellulaire immortalisée de fibroblastes humains selon la revendication 1, **caractérisée en ce qu'**elle est produite sans utiliser de cellules nourricières.

5. Procédé d'obtention de fibroblastes humains immortalisés tels que décrits dans l'une quelconque des revendications 1 à 4, comprenant les étapes suivantes :
- utiliser des fibroblastes primaires humains isolés de la peau saine d'un individu atteint de NBCCS ou du syndrome de Gorlin,
- immortaliser une lignée cellulaire par transduction rétrovirale avec un rétrovirus exprimant les protéines E6 et E7 de HPV16,
- sélectionner l'expression de la lignée cellulaire avec un milieu de sélection,
- vérifier l'atténuation de l'expression de p53, et
- vérifier des propriétés de croissance.

6. Procédé de criblage de médicament, dans lequel une lignée cellulaire immortalisée de fibroblastes humains selon l'une quelconque des revendications 1 à 5 est utilisée.

7. Procédé de criblage de médicament selon la revendication 6, comprenant les étapes suivantes de :
a. mise en contact d'un échantillon de lignée cellulaire immortalisée de fibroblastes humains selon l'une quelconque des revendications 1 à 4 avec un ou plusieurs composés test ou avec un mélange de composés,
b. mesure de l'expression ou de l'activité du gène rapporteur GLI1, et
c. sélection des composés pour lesquels une modulation de l'expression du gène rapporteur GLI1 est mesurée en b) par rapport à l'expression en l'absence de tout composés test ou mélanges de composés test.

8. Procédé de criblage de médicament selon la revendication 7, dans lequel le médicament identifié est un médicament antitumoral.

9. Méthode *in vitro* de criblage de composés candidats pour une utilisation pour le traitement préventif et/ou curatif du cancer de la peau, comprenant les étapes suivantes de :
a. mise en contact d'une lignée cellulaire immortalisée de fibroblastes humains selon l'une quelconque des revendications 1 à 4 avec un ou plusieurs composés test ou avec un mélange de composés,
b. mesure de l'expression ou de l'activité du gène rapporteur GLI1, et
c. sélection des composés pour lesquels une modulation de l'expression ou de l'activité du gène rapporteur GLI1 est mesurée en b) par rapport à l'expression ou à l'activité en l'absence de tout composés test ou mélanges de composés test.
